# EUROPÄISCHE PATENTSCHRIFT

(11) **EP 1 458 433 B1**
(45) Veröffentlichungstag und Bekanntmachung des Hinweises auf die Patenterteilung: **12.05.2010**
(21) Anmeldenummer: 02805294.2
(22) Anmeldetag: 27.11.2002
(51) Int. Cl.: A61M 1/16

(54) **BEHÄLTER ENTHALTEND MINDESTENS ZWEI FESTSTOFFE UND DESSEN VERWENDUNG**
CONTAINER CONTAINING AT LEAST TWO SOLID MATERIALS, AND USE THEREOF
RECEPTACLE CONTENANT AU MOINS DEUX MATIERES SOLIDES ET SON UTILISATION

(30) Priorität: 20.12.2001 DE 10162959
(43) Veröffentlichungstag der Anmeldung: 22.09.2004
(73) Patentinhaber: Fresenius Medical Care Deutschland GmbH, 61352 Bad Homburg v.d.H. (DE)
(72) Erfinder: DUMONT D'AYOT, François, F-69005 Lyon (FR); DUPLANIL, Stéphany, F-69005 Lyon (FR); GRAF, Thomas, 61352 Bad Homburg (DE); LAFFAY, Philippe, F-69110 St. Foy les Lyon (FR); WILD, Thomas, 66606 St. Wendel (DE)
(74) Vertreter: Dreyhsig, Jörg
(86) Internationale Anmeldenummer: PCT/EP2002/013334
(87) Internationale Veröffentlichungsnummer: WO 2003/053497

(56) Entgegenhaltungen:
- WO-A-00/57833
- WO-A-01/60428
- WO-A-90/13323
- US-A- 3 669 880
- US-A- 5 616 305
- PATENT ABSTRACTS OF JAPAN vol. 1997, no. 06, 30. Juni 1997 (1997-06-30) & JP 09 040562 A (TOMITA SEIYAKU KK), 10. Februar 1997 (1997-02-10)

## Beschreibung

Die Erfindung betrifft einen Behälter gemäß Anspruch 1. Ein Behälter gemäß dem Oberbegriff von Anspruch 1 ist aus der WO-A-90/13323 bekannt.

Zur Behandlung eines Patienten, der unter Niereninsuffiziens leidet, wird eine Dialyse durchgeführt. Dies erfolgt entweder im Peritoneum oder durch eine extrakorporale Dialyse oder Filtration des Blutes. Beiden Verfahren ist gemeinsam, daß Dialysierflüssigkeiten oder Dialysate die Abbaustoffe des Stoffwechsels aufnehmen. Diese Dialysate enthalten üblicherweise einen hohen Gehalt an Natriumchlorid und anderen Elektrolyten wie Calcium- oder Kaliumchlorid, eine Puffersubstanz wie beispielsweise Bicarbonat oder Acetat sowie Säure zur Herstellung eines physiologischen pH-Wertes und optional Glucose als Osmotikum.

Entweder werden fertige Dialysate angeliefert oder vor Ort Dialysate aus Konzentraten - auch Feststoff-Konzentraten - hergestellt. Dabei bieten Feststoffe den Vorteil des kleinen Packungsvolurnens und geringen Gewichtes. Obgleich die Feststoffe auch Nachteile aufweisen - beispielsweise sind die Elektrolytsalze stark hygroskopisch - ist die Tendenz dahingehend ausschließlich nur noch Feststoffkomponenten zur Dialysatherstellung anzubieten.

Die Feststoffe sind in der Regel Salze oder Kristalle, die in Pulverform oder in Granulatform vorliegen können. In der EP 0 287 987 ist ein Granulatgemisch aus den ionischen Komponenten, die für die Zubereitung von Dialysat notwendig sind. In der DE 43 03 372 ist ein Beutel zur Aufnahme von festem oder flüssigem Konzentrat üblicherweise Bicarbonat beschrieben, der zur Herstellung von Dialysat verwendet wird und direkt an eine Dialysemaschine angekoppelt wird.

Dabei werden nur Komponenten zusammen in einem Behälter gelagert, die während ihrer Lagerzeit nicht miteinander reagieren oder sich in ihren chemischen und/oder physikalischen Eigenschaft beeinflussen, also Stoffe, die miteinander kompatibel sind. Stoffe, die sich in ihren chemischen und/oder physikalischen Eigenschaften unter Umständen beinflussen, nennt man nicht miteinander kompatible Stoffe. Die mangelnde Kompatibilität muß nicht spontan eintreten, sondern kann auch eintreten bei einem Verfahren, dem die Komponenten üblicherweise unterzogen werden, wie die Sterilisation von Dialysaten.

Zur Überwindung dieses Problems ist die getrennte Lagerung vorgeschlagen worden, sei es durch voneinander losgelöste Behälter oder Behälter mit mehreren Kammern, um erst kurz vor Behandlung die Durchmischung der Stoffe durchzuführen. Allerdings ergeben sich dadurch andere Schwierigkeiten, wie die problematische Fertigung eines Konnektionssystems zwischen den sterilen Behältern oder der einzelnen Behälter an die Dialysemaschine, sei es durch Zusammenstecken von zueinander passenden Schlüssel-Schloß-Konnektionssystemen, durch Aufbrechen eines Verbindungskanals oder Aufreißen einer peelbaren Naht. Darüber hinaus ist es möglich, daß die Zusammenführung der Einzelkomponenten nicht vollständig erfolgt bzw. nur eine der Komponenten verabreicht wird.

Es ist daher die Aufgabe der vorliegenden Erfindung, einen Behälter bereitzustellen, der die im Stand der Technik bekanntgewordenen Nachteile vermeidet. Die Aufgabe wird durch die Merkmale des ersten Anspruchs gelöst.

Kennzeichnend für die Erfindung ist die schichtweise Lagerung mindestens zweier Feststoffe innerhalb eines Behälters. Die Feststoffe sollen Komponenten für ein Dialysat darstellen, liegen dann als Pulver, Kristall oder Granulat vor, da die Dialysatkomponenten aus Salzen oder Glucose hergestellt werden. Die beinhalteten Feststoffe sind wasserlöslich Ein derartiger Behälter wird für die Herstellung eines Diaysates verwendet.

Die Stoffschichten, die nicht miteinander kompatibel sind, sind erfindungsgemäß voneinander beabstandet. Die miteinander inkompatiblen Komponenten werden durch eine Schicht einer weiteren, dritten Komponente voneinander getrennt, die selbst mit jedem der beiden anderen Komponenten kompatibel ist.

Dabei wird kompatibel definiert als sich gegenseitig nicht in ihren chemischen und/oder physikalischen Eigenschaften zu verändern, inkompatibel oder nicht kompatibel entsprechend umgekehrt. Darunter wird verstanden daß der Behälter auch über eine längere Lagerzeit hinweg oder extremen Umgebungsbedingungen wie etwa bei einer Sterilisation ausgesetzt ist.

Der dritte Feststoff ist eine weitere Komponente zur Herstellung eines Dialysates, der als Granulat, Pulver oder Kristall vorliegt und ebenfalls wasserlöslich ist.

Beispiele für Komponenten, die zur Herstellung von Dialysat verwendet werden, sind Elektrolyte, Säuren, Natriumchlorid, Natriumbicarbonat und Glucose. Von diesen Stoffen sind beispielsweise Natriumbicarbonat und Glucose nicht miteinander kompatibel. Daher wird erfindungsgemäß eine Schicht aus Natriumchlorid zwischen der Schicht aus Natriumbicarbonat und der Schicht aus Glucose eingefüllt.

Als Behälter ist jede Art von fester Kartusche oder flexiblem Beutel denkbar, der aus einem Material hergestellt werden sollte, der mit seinen Inhaltsstoffen ebenfalls kompatibel ist. Im Bereich der Medizintechnik wurden früher hauptsächlich feste Kartuschen aus Polycarbonat oder flexible Beutel aus Polyvinylpyrrolidon bereitgestellt. Diese werden jedoch mehr und mehr durch Polyolefine, insbesondere Polypropylen und Polyethylen in Verbindung mit synthetischen Kautschuken abgelöst. Besondere Beschichtungen, die zusätzlich eine Gasbarriere oder Wasserdampfbarriere, besondere Kompatibilitäten oder ähnliche Vorteile bieten, sind im Stand der Technik zahlreich beschrieben.

Der Behälter sollte mindestens einen Einlauf besitzen, um ihn mit den Komponenten füllen zu können. Dieser Einlauf kann erfindungsgemäß auch gleichzeitig als Auslauf genutzt werden. Jedoch ist auch denkbar, Ein- und Ausläufe getrennt vorzusehen und/ oder mehrere Einläufe bzw. Ausläufe vorzusehen. Auch können diese Behälter zusätzlich Anschlüsse wie Zuspritzstellen, Be- und Entlüftungsvorrichtungen oder ähnliches aufweisen.

Die Feststoffe werden erfindungsgemäß schichtweise in den Behälter gefüllt. Damit beim Transport die Schichten nicht verrutschen, ist es vorteilhaft die Schichten zu fixieren. Dabei ist denkbar, eine Abdeckplatte auf die Feststoffschichten zu pressen, die arretierbar ist. Besonders vorteilhaft ist es jedoch, nicht benötigte Gase oder Flüssigkeiten aus dem Behälter zu entfernen, beispielsweise durch Absaugen.

Aus diesem Grund ist die bevorzugte Ausführungsform der Erfindung ein flexibler Beutel, in dem nach dem schichtweise Einfüllen der Feststoffe mit Hilfe einer Pumpe ein Vakuum gezogen wird und der anschließend luftdicht verschlossen wird. Dabei ist zu beachten, daß nie ein vollständig luftleerer Raum entstehen kann, weswegen die korrekte Beschreibung einen Innenraum des Behälters bezeichnet, der möglichst wenig Gas oder Flüssigkeit enthält. Ebenso ist die Ausführungsform als flexibler Beutel nicht unbedingt notwendig, doch besonders hilfreich, will man mit Hilfe eines "Vakuumpacks" eine Fixierung der beinhalteten Feststoffe erreichen.

Die Fixierung der Feststoffe an ihrem Ort innerhalb des Beutels dient dazu, ein Durchmischen zu vermeiden, wodurch dann nicht kompatible Feststoffe miteinander in Berührung kommen könnten. Auch wenn die Erfindung nicht auf die Verwendung von Feststoffen zur Herstellung eines Dialysates beschränkt ist, so ist die bevorzugte Ausführungsform doch ein flexibler Behälter, der mit möglichst allen zur Herstellung eines Dialysates notwendigen Stoffe gefüllt ist, mindestens aber mit Feststoffen gefüllt, die ein Teilkonzentrat bilden.

Ein übliches Teilkonzentrat ist die Verwendung von den drei Stoffen Bicarbonat, Natriumchlorid und Glucose. Glucose unterliegt einem Abbau, wenn in Kontakt mit einem inkompatiblen Stoff basichen Charakters kommt, hier Bicarbonat. Dies ist verstärkt während der Heißdampfsterilisation der Fall. Werden zwei inkompatible Stoffe, wie diesem Fall Natriumbicarbonat und Glucose, für das Teilkonzentrat verwendet, so ist die besonders bevorzugte Ausführungsform ein flexibler Beutel mit drei Schichten Feststoffe aus Glucose, Natriumchlorid und Natriumbicarbonat darstellt, wobei Natriumchlorid die mittlere Grenzschicht bildet und wobei die Fixierung der Schichten durch das Absaugen der sich im Inneren befindlichen Luft gebildet wird.

Ein derartig gefüllter Beutel dient als Konzentratbereitstellung zur Herstellung von Dialysat. Besonders vorteilhaft ist es, wenn die im Beutel vorgelegte Menge gerade ausreichend ist, um genau die Menge an Dialysat herzustellen, die für eine Dialyse notwendig und ausreichend ist.

## Patentansprüche

1. Behälter mit mindestens einem Ein- oder Auslaß enthaltend mindestens zwei voneinander verschiedene wasserlösliche Feststoffe, die sich in ihren chemischen und/oder physikalischen Eigenschaften beeinflussen, zur Herstellung eines Dialysats, **dadurch gekennzeichnet, dass** die mindestens zwei voneinander verschiedenen Feststoffe in Schichten angeordnet und durch eine Schicht eines dritten wasserlöslichen Feststoffes voneinander beabstandet sind, der mit den mindestens zwei voneinander verschiedenen Feststoffen nicht zu einer Beeinflussung der chemischen und/oder physikalischen Eigenschaften der Feststoffe führt, wobei die Feststoffe Pulver, Kristalle oder Granulate sind.

2. Behälter nach Anspruch 1, **dadurch gekennzeichnet, dass** die wasserlöslichen Feststoffe aus der Gruppe Elektrolyte, Säuren, Natriumchlorid, Natriumbicarbonat und Glucose ausgewählt sind.

3. Behälter nach Anspruch 2, **dadurch gekennzeichnet, dass** der dritte Feststoff Natriumchlorid ist.

4. Behälter nach einem der Ansprüche 1 bis 3, **dadurch gekennzeichnet, dass** der Behälter zur Fixierung der beinhalteten Feststoffe als Vakuumpack ausgeführt ist.

5. Behälter nach einem der Ansprüche 1 bis 4, **dadurch gekennzeichnet, dass** der Behälter flexibel ist.

## Claims

1. A container comprising at least one inlet or outlet and containing at least two different water-soluble solids mutually affecting their chemical and/or physical properties for producing a dialysate, **characterized in that** the at least two different solids are arranged in layers and spaced apart by a layer of a third water-soluble solid that with the at least two different solids does not lead to an affection of the chemical and/or physical properties of the solids, the solids being powders, crystals or granulate materials.

2. A container according to claim 1, **characterized in that** the water-soluble solids are selected from the group of electrolytes, acids, sodium chloride, sodium bicarbonate and glucose.

3. A container according to claim 2, **characterized in that** the third solid is sodium chloride.

4. A container according to any of claims 1 to 3, **characterized in that** the container is designed as vacuum packaging to fix the contained solids.

5. A container according to any of claims 1 to 4, **characterized in that** the container is flexible.

## Revendications

1. Récipient pourvu d'au moins une entrée ou une sortie contenant au moins deux matières solides hydrosolubles qui sont différentes l'une de l'autre et s'influencent l'une l'autre en ce qui concerne leurs propriétés chimiques et/ou physiques, afin de préparer un dialysat, **caractérisé en ce que** lesdites au moins deux différentes matières solides sont disposées en couches et tenues à distance l'une de l'autre par une couche d'une troisième matière hydrosoluble qui, avec les lesdites au moins deux différentes matières solides, ne viendra pas influencer les propriétés chimiques et/ou physiques des matières solides, les matières solides étant des poudres, des cristaux ou des granulés.

2. Récipient selon la revendication 1, **caractérisé en ce que** lesdites matières solides hydrosolubles sont choisies dans le groupe des électrolytes, des acides, du chlorure de sodium, du bicarbonate de sodium et du glucose.

3. Récipient selon la revendication 2, **caractérisé en ce que** la troisième matière solide est du chlorure de sodium.

4. Récipient selon l'une des revendications 1 à 3, **caractérisé en ce que** ledit récipient est réalisé en tant qu'emballage sous vide afin de fixer les matières solides qu'il contient.

5. Récipient selon l'une des revendications 1 à 4, **caractérisé en ce que** ledit récipient est flexible.
